# EUROPEAN PATENT SPECIFICATION

(11) **EP 2 944 323 B1**
(45) Date of publication and mention of the grant of the patent: **27.03.2019**
(21) Application number: 13870762.5
(22) Date of filing: 11.01.2013
(51) Int. Cl.: C07K 16/28, A61P 35/00, A61N 5/10, A61K 39/395

(54) **AGENTS FOR TREATING TUMOURS, USE AND METHOD THEREOF**
MITTEL ZUR BEHANDLUNG VON TUMOREN, VERWENDUNG UND VERFAHREN DAFÜR
AGENTS POUR TRAITER DES TUMEURS ET UTILISATION ET MÉTHODES CORRESPONDANTES

(43) Date of publication of application: 18.11.2015
(73) Proprietor: Dingfu Biotarget Co., Ltd, Suzhou, Jiangsu 215125 (CN)
(72) Inventor: FU, Yangxin, Chicago, IL 60637 (US)
(74) Representative: Weickmann & Weickmann PartmbB
(86) International application number: PCT/CN2013/070342
(87) International publication number: WO 2014/107873

(56) References cited:
- WO-A1-2009/089149
- WO-A1-2009/089149
- WO-A2-2012/177624
- BAZALOVA MAGDALENA ET AL: "Modality comparison for small animal radiotherapy: A simulation study", MEDICAL PHYSICS, AIP, MELVILLE, NY, US, vol. 41, no. 1, 1 January 2014 (2014-01-01), XP012184204, ISSN: 0094-2405, DOI: 10.1118/1.4842415 [retrieved on 1901-01-01]
- WEIQING JING ET AL: "Combined immune checkpoint protein blockade and low dose whole body irradiation as immunotherapy for myeloma", JOURNAL FOR IMMUNOTHERAPY OF CANCER, BIOMED CENTRAL LTD, LONDON, UK, vol. 3, no. 1, 20 January 2015 (2015-01-20), page 2, XP021210655, ISSN: 2051-1426, DOI: 10.1186/S40425-014-0043-Z
- TENG FEIFEI ET AL: "Radiotherapy combined with immune checkpoint blockade immunotherapy: Achievements and challenges", CANCER LETTERS, vol. 365, no. 1, 2015, pages 23-29, XP029222836, ISSN: 0304-3835, DOI: 10.1016/J.CANLET.2015.05.012
- DAVID KILLOCK: "Immunotherapy: Combined immunoradiotherapy reinvigorates antitumour immunity", NATURE REVIEWS CLINICAL ONCOLOGY, vol. 12, no. 6, 24 March 2015 (2015-03-24) , pages 311-311, XP055277370, NY, US ISSN: 1759-4774, DOI: 10.1038/nrclinonc.2015.54
- YURU MENG ET AL.: 'Radiation-inducible Immunotherapy for Cancer: Senescent Tumor Cells as a Cancer Vaccine' MOLECULAR THERAPY vol. 20, no. 5, May 2012, pages 1046 - 1055, XP055261840
- CHARLES J ROSSER ET AL: "Molecular fingerprinting of radiation resistant tumors: Can we apprehend and rehabilitate the suspects?", BIOMED CENTRAL, vol. 9, no. 225, 9 July 2009 (2009-07-09), pages 1-10,
- CARRIE PRINTZ: "Radiation oncologists work to protect patients'", CANCER, 1 July 2012 (2012-07-01), pages 3223-3225,
- FRANCESCA DE BACCO ET AL: "Induction of MET by ionizing radiation and its role in radioresistance and invasive growth of cancer", J NATL CANCER INST, vol. 103, 4 April 2011 (2011-04-04), pages 645-661,

## Description

### FIELD OF THE INVENTION

The present disclosure relates to the field of biomedicine, specifically, it relates to agents for treating and/or preventing resistance of tumor cells to radiation therapy (RT), the use and relevant method thereof.

### BACKGROUND OF THE INVENTION

The mechanism of radiation therapy is based on the cause of lethal DNA damage to tumor cells or tumor-associated mesenchyme. Cancer patients generally receive low dosage (1.5-3Gy) RT (in a few times) daily for several weeks, which is often combined with chemotherapy. Traditionally, RT is considered to be immunosuppressive²⁰. Although some studies investigated the potential immunoregulatory effects of local RT on tumors, there were contradictory reports as to whether these responses promote or interfere with tumorregression²¹⁻²⁴.

Recent studies of the inventor show that local ablative RT can induce immune responses leading to tumor regression^{1,2}. However, tumor recurrence after RT is a common clinical problem after the initial response, i.e., there are RT-resistant tumors and/or tumor cells. In the present disclosure, the inventor demonstrates that a B7-H1 blocking antibody can act synergistically with RT, the corresponding use leading to the regression of tumors, particularly RT-resistant tumors.

Radiation can cause innate and adaptive immune responses. Tumor cells exposed to radiation are capable of secreting danger signals (e.g., HMGB-1), which crosstalk with TLR4 expressed on dendritic cells and subsequently promote cross-presentation of antigens³. In addition, previous results of the inventor show that type I interferon triggered by radiation can enhance danger signaling and increase the cross-priming of CD8⁺ T-cells². Subsequently, dendritic cells and macrophages that can ingest apoptotic tumor cells and migrate into lymph nodes and activate T-cells⁴. During these processes, co-stimulatory molecules increase T-cell responses and result in tumor shrinkage. In contrast, co-inhibitory molecules suppress T-cell responses and contribute to tumor evasion⁵. However, it is still not clear which co-signaling molecules (e.g., co-inhibitory molecules) are up-regulated and inhibit T-cell immunity after RT.

B7-H1/PD1 pathway plays a role in inhibition of tumor immune.
B7-H1 is a co-inhibitory molecule and a member of the B7 family, and can be inducibly expressed on tumor cells, dendritic cells and macrophages⁶. Tumor-associated B7-H1 is involved in the induction of apoptosis of tumor-reactive T-cells and the impairment of cytotoxicity of CTLs⁷. Furthermore, B7-H1 expressed on dendritic cells is considered to inhibit the proliferation of T-cells and prevent T-cells from producing cytokines⁸. PD-1 is an inhibited receptor inducibly expressed on activated T-cells, and can promote anergy, apoptosis and exhaustion of T-cells⁵. PD-1 signaling is considered as a regulator of antigen-specific T-cell exhaustion in chronic infections (e.g., LCMV in mice and HIV and HCV in humans)⁹⁻¹². The hallmark of exhausted T-cells includes impaired proliferation and effector function¹³. Several references have implicated that B7-H1/PD1 pathway contributes to T-cell exhaustion in cancers¹⁴⁻¹⁶. Blocking of B7-H1/PD1 signaling has been shown to be able to restore functional T-cell responses and delay tumor growth^{14, 16, 17}. As illustrated in WO 2012/177624 A2 describing the combined use of a PD-1 antibody with radiation therapy, these evidences suggest that blocking of B7-H1/PD-1 signaling may be of important value for designing a combined administration of agents and radiation therapy.

### SUMMARY OF THE INVENTION

The studies of the inventor have shown that a recently developed regimen, i.e., local high-dosage radiation therapy, can reduce tumor burden and increase antitumor immunity. However, tumor recurrence often occurs, i.e., RT-resistant tumor cells or tissues exist after radiation therapy. These tumor cells or tissues can further develop into tumors or have the potential to develop into tumors. The present inventor surprisingly discovers that RT can induce both the expression of B7-H1 on tumors and the expression of PD-1 on T-cells, and the expression of both B7-H1 and PD-1 can inhibit further antitumor immunity and thus cause tumor recurrence. However, the RT-induced immunosuppression can be reduced (i.e., the resistance of tumor cells to RT can be reduced) and the antitumor immunity of hosts can be enhanced by using an immunotherapy and/or product that block B7-H1/PD1 signaling after RT. Therefore, the approach chosen in the present disclosure presents a novel strategy/regimen, i.e., radiation therapy followed by timely administration of an immunotherapy and/or product that block B7-H1/PD1 signaling (i.e., an anti-B7-H1 antibody) can enhance antitumor therapeutic activities and achieve beneficial therapeutic effects for cancer patients.

Accordingly, in one aspect, the present disclosure relates to a B7-H1 blocking antibody for use in a method of treating cancer in combination with a radiation treatment, wherein the B7-H1 blocking antibody is administered following the radiation treatment, and wherein said B7-H1 blocking antibody is an anti-B7-H1 antibody.

In a specific embodiment, the antibody can be a blocking monoclonal antibody to B7-H1, e.g., a monoclonal antibody 10F.9G2 commercially available from Bio-X cell (West Lebanon, NH03784, USA).

In some embodiments of the present disclosure, the radiation therapy is a single or multiple X-ray irradiation, e.g., 1, 2, 3, 4, 5, 6 or more X-ray irradiations. Preferably, X-ray dosage used in each irradiation may be 5-20Gy, such as 5-8, 5-12 or 5-15Gy. In particular, when multiple X-ray irradiations are administered, the interval between irradiations may be one to several hours (e.g., 2, 3, 4, 5, 6, 7, 8, 9, or up to 24 hours), one to several days (e.g., 2, 3, 4, 5, 6, 7, 8, 9, 10, 11, 12, 13, 14, 15, 16, 17, 18, 19 days, etc.).

In other embodiments, the tumor may be, but is not limited to, breast cancer, ovarian cancer, bladder cancer, lung cancer, prostate cancer, pancreatic cancer, colon cancer or melanoma and/or cells thereof, for example, breast cancer cells (such as TUBO cells) or Myc-Cap tumor cell line.

In certain embodiments, the composition further comprises an additional compound that can be used as a chemotherapeutic agent. In particular, the additional compound may include, but not limited to, adriamycin, cyclophosphamide and Taxanes [paclitaxel (Taxol) and docetaxel (Taxotere)], capecitabine (Xeloda), gemcitabine (Gemzar), vinorelbine (Navelbine), tamoxifen, aromatase inhibitors (Arimidex, Femara, and Aromasin), 5-FU with folinicAcid, irinotecan (Camptosar), oxaliplatin, cisplatin, carboplatin, estramustine, mitoxantrone (Novantrone), prednisone, vincristine (Oncovin), etc.

In other embodiments, the antibody may be provided in form of a composition suitable for direct administration at tumor sites or suitable for systemic administration.

In some embodiments, the B7-H1 blocking antibody for use in the present disclosure is administered within several hours (e.g., 10-48 hours), several days (e.g., 1, 2, 3, 4, 5, 6, 7, 8 or 9 days) or several weeks (e.g., 1-8, 1-7, 1-6, 1-5, 1-4, 1-3 or 1-2 weeks) after radiation therapy or radiation treatment. When desired, the antibody can be administered for multiple times, for example, 2, 3, 4 ,5, 6 or more times, and the time interval between two administrations can be several hours, one day, several days (e.g., 2-30, 2-25, 2-20, 2-15, 2-14, 2-13, 2-12, 2-11, 2-10, 2-9, 2-8, 2-7, 2-6, 2-5, 2-4, or 2-3 days).
In yet other embodiments, the antibody may be administered systemically or merely at a site of tumor.
In certain embodiments, the B7-H1 blocking antibody to be administered may further comprise an additional compound that can be used as a chemotherapeutic agent. In particular, the additional compound may include, but not limited to, adriamycin, cyclophosphamide and Taxanes [paclitaxel (Taxol) and docetaxel (Taxotere)], capecitabine (Xeloda), gemcitabine (Gemzar), vinorelbine (Navelbine), tamoxifen, aromatase inhibitors (Arimidex, Femara, Aromasin), 5-FU withfolinic Acid, irinotecan (Camptosar), oxaliplatin, cisplatin, carboplatin, estramustine, mitoxantrone (Novantrone), prednisone, vincristine (Oncovin), etc. Alternatively, the method as described in the present disclosure can be used in combination with an additional method for treating and/or preventing a tumor (e.g., surgery, chemotherapy or radiation therapy).

### BRIEF DESCRIPTION OF THE DRAWINGS

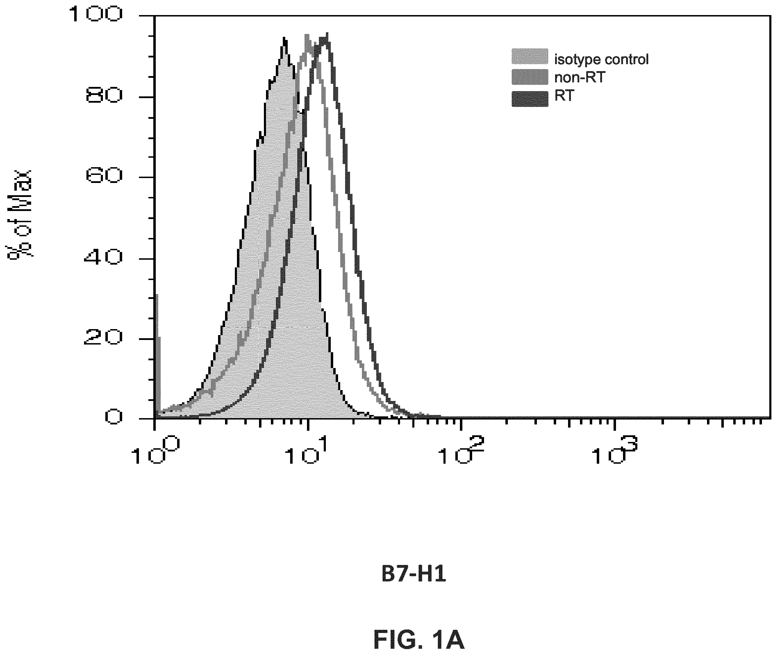

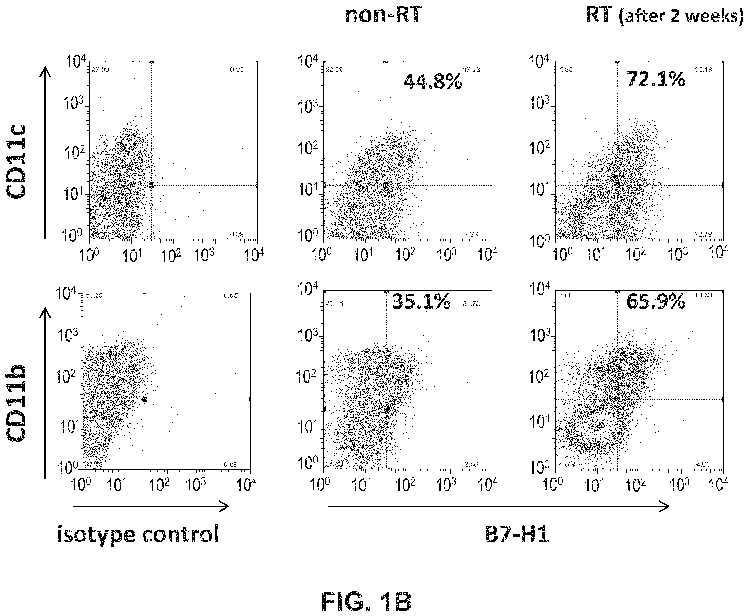

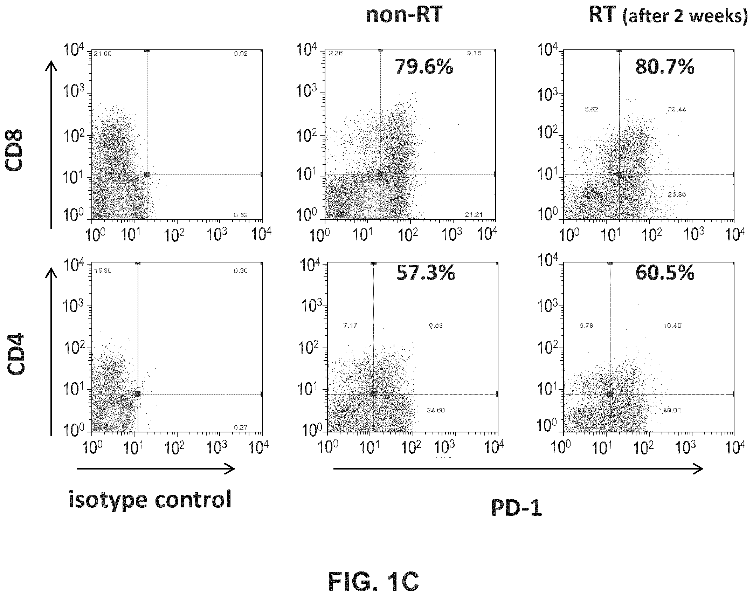

**FIG. 1****:** The expression of B7-H1 and PD-1 in tumor microenvironment after radiation.
   5×10⁵ TUBO cells were injected subcutaneously in the flanks of Balb/c mice. At day 14, the mice were locally irradiated with 15 Grays (Gy). At day 28, the tumors were removed and digested to obtain a single-cell suspension for staining. (A) The expression of B7-H1 on tumor cells was affected by the radiation; (B) the expressions of B7-H1 on DCs and macrophages were changed after the radiation; (C) PD-1 was highly expressed on both irradiated and unirradiated T-cells.

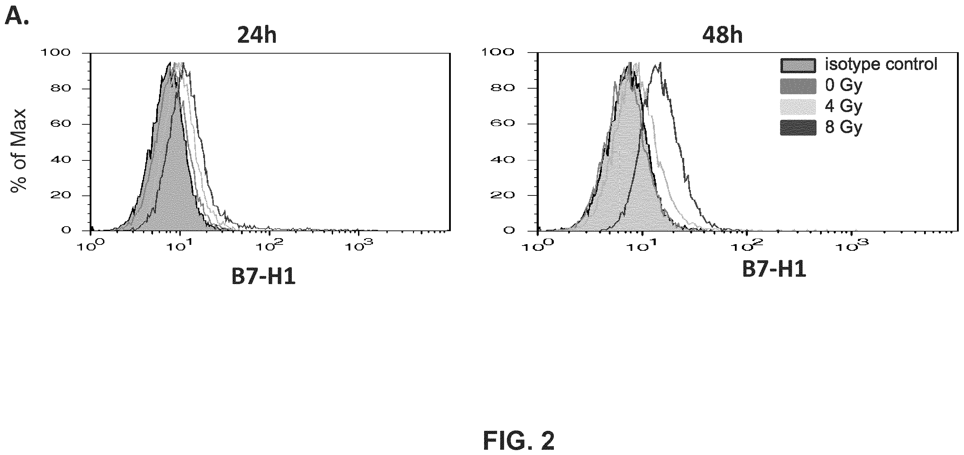

**FIG. 2****:** RT causes up-regulation of B7-H1 on Myc-Cap cell line. A. The expression of B7-H1 after RT. The tumor cells (Myc-Cap tumor cell line) were treated with 0, 4 and 8 Gy of irradiation. Then, the irradiated tumor cells were cultured for 24 or 48 hours. After 24 or 48 hours, the cells were harvested, and then stained with an anti-B7-H1 monoclonal antibody. The unirradiated Myc-Cap cells were used as a control.
**FIG. 3****:** Blockade of B7-H1 improves radiation therapy.
   5×10⁵ TUBO cells were injected subcutaneously in the flanks of Balb/c mice. At day 14, the mice were locally treated with a dosage of 12 Gy of irradiation. At days 15, 18 and 21, the mice were injected intraperitoneally with 50 µg of a B7-H1 blocking monoclonal antibody (clone 10F.9G2). Tumor growth was monitored.

### DETAILED DESCRIPTION OF THE INVENTION

As used herein, the "agent capable of inhibiting B7-H1/PD1 signaling" is a blocking antibody to B7-H1. In a specific embodiment, the blocking antibody can be a specific monoclonal antibody, polyclonal antibody, humanized antibody, chimeric antibody or an antigen-specific fragment (e.g., Fab, Fv, ScFv antibody fragment, or the like) thereof. A person skilled in the art will appreciate that any antibody able to specifically bind to B7-H1 protein and influence the function and/or structure thereof may potentially act as the "agent capable of inhibiting B7-H1/PD1 signaling" of the present disclosure.

As used herein, the term "radiation therapy" or "radiation treatment" includes, for example, fractionated radiation therapy, non-fractionated radiation therapy and super-fractionated radiation therapy, as well as a combination of radiation and chemotherapy. The type of radiation may further include ionizing (y) radiation, particle radiation, low energy transfer (LET), high energy transfer (HET), X-ray radiation, UV radiation, infrared radiation, visible light, photosensitizing radiation, etc.

In one embodiment of the present disclosure, the radiation therapy or radiation treatment is a single or multiple X-ray irradiations, e.g., 1, 2, 3, 4, 5, 6 or more X-ray irradiations. Preferably, the dosage of X-ray used in each irradiation may be 5-20 Gy, such as 5-8, 5-12 or 5-15 Gy. In particular, when multiple X-ray irradiations are administered, the interval between irradiations may be one to several hours (e.g., 2, 3, 4, 5, 6 ,7, 8, 9 or up to 24 hours), one to several days (e.g., 2, 3, 4, 5, 6 ,7 ,8, 9, 10, 11, 12, 13, 14, 15, 16, 17, 18, 19 days, etc.).

As used herein, the terms "chemotherapeutic agent", "chemical therapeutic agent" and "agent for chemotherapy" can be used interchangeably. It includes a composition comprising a single active ingredient or a combination of multiple chemotherapeutic agents. In a subject in need of a therapy, chemotherapy can be used in combination with surgery or radiation therapy, or with other forms of anti-tumor therapies, for example, the "agent capable inhibiting B7-H1/PD1 signaling" of the present disclosure. In particular, the chemotherapeutic agent includes, but not limited to, adriamycin, cyclophosphamide and Taxanes [paclitaxel (Taxol) and docetaxel (Taxotere)], capecitabine (Xeloda), gemcitabine (Gemzar), vinorelbine (Navelbine), tamoxifen, aromatase inhibitors (Arimidex, Femara, Aromasin), 5-FU withfolinic Acid, irinotecan (Camptosar), oxaliplatin, cisplatin, carboplatin, estramustine, mitoxantrone (Novantrone), prednisone, vincristine (Oncovin),etc., or a combination thereof.

In the context of the present disclosure, the term "resistance to radiation therapy" means that cells (e.g., tumor cells) do not lose their ability to continue to propagate and/or grow after being through radiation therapy or treatment. Generally, such resistance can lead to decrease or loss of the efficacy of the radiation therapy in tumor treatment, which in turn can cause tumor recurrence.

In the context of the present disclosure, the term "tumor", "cancer" or "hyper proliferative disease" refers to the growth and proliferation of any malignant or benign cancerous cells, including all transformed cells and tissues and all cancerous cells and tissues.

Examples of the cancer include, but not limited to, carcinoma, lymphoma, blastocytoma, sarcoma, leukemia or malignant lymphatic tumors. Specific examples of the cancer include squamous cell cancer (e.g., epithelium squamous cell cancer), lung cancer, including small cell lung cancer, non-small cell lung cancer, lung adenocarcinoma and squamous cell cancer of the lung, peritoneal cancer, hepatocellular cancer, gastric cancer, including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial cancer or uterine cancer, salivary gland cancer, kidney cancer, prostate cancer, vulvar cancer, thyroid cancer, liver cancer, anus cancer, penis cancer, and head and neck cancer. Further examples of the cancer are listed in the elsewhere of the present disclosure. In particular, the tumor is selected from: breast cancer, ovarian cancer, bladder cancer, lung cancer, prostate cancer, pancreatic cancer, colon cancer and melanoma and/or cells thereof, for example, breast cancer cells (such as TUBO cells) or Myc-Cap tumor cell line.

In some embodiments of the present disclosure, the agent capable of inhibiting B7-H1/PD1 signaling of the present disclosure or the composition comprising the agent is administered within several hours (e.g., 10-48 hours), several days (e.g., 1, 2, 3, 4, 5, 6, 7, 8 or 9 days) or several weeks (e.g., 1-8, 1-7, 1-6, 1-5, 1-4, 1-3 or 1-2 weeks) after radiation therapy or radiation treatment. When desired, the agent or the composition can be administered for multiple times, for example, 2, 3, 4 ,5, 6 or more times, and the interval between administrations can be several hours, one day, several days (e.g., 2-30, 2-25, 2-20, 2-15, 2-14, 2-13, 2-12, 2-11, 2-10, 2-9, 2-8, 2-7, 2-6, 2-5, 2-4, or 2-3 days).

For the prevention or treatment of resistance to radiation therapy, the dosage and manner for administrating the agent or composition of the present disclosure can be decided by physicians according to criteria known in the art. The administered concentration and dosage of the agent that inhibits B7-H1/PD1 signaling may depend on the type of the cancer to be treated, the severity and course of the disease, the size of the tumor, the degree of metastasis, the aim of administration is preventive or therapeutic, previous therapy, the patient's medical history and response to antibodies, and the discretion of the attending physician. For repeated administration over several days or longer, depending on the condition, the treatment can be maintained until a desired suppression of the symptoms is achieved, for example, a reduction of tumor size/volume and reduction of metastasis. The course of treatment can be monitored by a conventional method or analysis based on the criteria known to physicians or any person skilled in the art.

Specifically, for an antibody, the dosage to be administered may range from 0.1 to 100 mg/kg of patient's body weight, e.g., 0.1 to 20 mg/kg of patient's body weight, or 0.1 to 10 mg/kg of patient's body weight. In general, human antibodies have longer half-life in humans than antibodies derived from other species due to immune responses to foreign polypeptides. Therefore, a lower dosage of a human antibody and a lower frequency of administration are generally possible. Furthermore, the dosage and frequency of administration of an antibody can be reduced by enhancing the uptake and the tissue penetration (e.g., into the brain) of the antibody through a modification such as lipidation.

The pharmaceutical composition according to the present disclosure can comprise a pharmaceutically acceptable excipient, carrier, buffering agent, stabilizer or other materials known to those skilled in the art. Such materials shall be non-toxic and shall not interfere with the efficacy of the active ingredient. Such materials may include any solvent, dispersion media, coating, antibacterial and antifungal agent, isotonic and absorption delay agent, physiologically compatible substance, etc. The pharmaceutically acceptable carrier can be, for example, water, saline, phosphate buffered saline, glucose, glycerol, ethanol or the like, and a combination thereof. In many cases, the pharmaceutical composition may comprise an isotonic agent, for example, sugar, a polyol such as mannitol, sorbitol, or preferably sodium chloride. The pharmaceutically acceptable substance can also be a wetting agent or a small amount of auxiliary substance, such as a moisturizer or an emulsifying agent, a preservative or a buffering agent, which can increase the shelf life or efficacy of the antibody. The concrete properties of the carrier or other materials will depend on the route of administration, which can be oral, topical, by inhalation or by injection, for example, intravenously. In one embodiment, the pharmaceutical composition is administered by intravenous infusion or injection. In another preferred embodiment, the pharmaceutical composition is administered by intramuscular or subcutaneous injection.

The pharmaceutical composition for oral administration may be in the form of tablet, capsule, powder or liquid, for example, comprising an inert diluent or an assimilable edible carrier. A tablet can comprise a solid carrier, for example, gelatin or an adjuvant. A liquid pharmaceutical composition typically comprises a liquid carrier, for example, water, petroleum, animal or vegetable oil, mineral oil or synthetic oil. It is possible to include physiological saline solution, glucose or other sugar solutions, or glycols such as ethylene glycol, propylene glycol or polyethylene glycol. A specifically binding member (and, when desired, other components) may also be encapsulated into hard or soft shell gelatin capsules, compressed into tablets, or incorporated directly into the diet of a subject. For oral therapeutic administration, the active ingredient may be blended with excipients, and can be used in the form of ingestible tablets, buccal tablets, troches, capsules, elixirs, suspensions, syrups, wafers or the like. In order to administer the product of the present disclosure in routes other than parenteral administration, it may be necessary to coat a compound with a material that can prevent its deactivation or co-administer them.

As to intravenous injection, or injection at a site of pain (e.g., tumor sites), the active ingredient will be in the form of a parenterally acceptable aqueous solution, which is pyrogen-free and has a suitable pK, isotonicity and stability. A person skilled in the art can readily use, for example, an isotonic vehicle such as sodium chloride solution, Ringer's injection, or lactated Ringer's injection to prepare a suitable solution. When desired, preservatives, stabilizers, buffering agents, anti-oxidants and/or other additives can be contained.

The agent or composition of the present disclosure can be used alone or in combination with an additional therapy simultaneously or sequentially, depending on the condition to be treated.

The following examples are merely intended to illustrate the present disclosure in further detail and should by no means be construed as limiting the scope of the invention. A person skilled in the art will appreciate that modifications can be made to the following embodiments without departing from the scope, spirit and principle of the present disclosure as claimed by the appended claims.

In the following examples, methods, apparatuses, reagents and protocols commonly used by those skilled in the art were employed, unless specified otherwise.

### Example 1. B7-H1 and PD-1 were highly expressed in tumor microenvironment after radiation.

Recurrence after RT is a common problem, which is at least partially due to the presence of RT-resistant tumor cells and/or tissues in a subject. The present inventor proposes that the course of recurrence, i.e., progression of these RT-resistant tumors, may involve inhibitory molecules that inhibit T-cell responses. In order to investigate whether or not RT induces the expression of B7-H1/PD1, the inventor stained B7-H1 on tumor cells, dendritic cells and macrophages, and stained PD-1 on CD4⁺ T-cells and CD8⁺T-cells. The experimental procedure was briefly described as follows. 5×10⁵ TUBO tumor cells (derived from breast cancer cells of Balb/c Her2/neu transgenic mice)¹⁸ were injected subcutaneously in the flanks of Balb/c mice. At day 14, the mice were locally irradiated with 15 Grays (Gy) using an X-ray generator (PCM 1000, Pantak). At day 28, the tumors were removed and digested for 30 min with 0.2 mg/ml of collagenase to obtain a single-cell suspension for staining B7-H1 with a standard staining protocol using 0.5 µg/ml of monoclonal antibody 10F.9G2 purchased from Bio-X cell, West Lebanon, NH03784, USA.

The inventor discovered that B7-H1 was expressed not only on the tumor cells but also on dendritic cells and macrophages after radiation **(****FIGS. 1A** and **1B****).** The inventor also discovered that PD-1 was highly expressed on infiltrative CD8⁺ T-cells and CD4⁺ T-cells **(****FIG. 1C****)**.

### Example 2. RT caused upregulation of B7-H1.

In order to investigate whether or not RT can induce expression of B7-H1 on tumor cells (Myc-Cap prostate cancer cell line¹⁹), the tumor cells (Myc-Cap tumor cell line) were treated with 0, 4 and 8 Gy of irradiation. Then, the irradiated tumor cells were cultured for 24 or 48 hours. After 24 or 48 hours, the cells were harvested, and the harvested cells (10⁶ cells) were subjected to standard staining with an anti-B7-H1 monoclonal antibody (0.5 µg/ml of antibody 10F.9G2, purchased from Bio-X cell, West Lebanon, NH03784, USA). The unirradiated Myc-Cap cells were used as a control. The results showed that RT significantly upregulated the expression of B7-H1 in Myc-Cap cells.

### Example 3.Blockade of anti-B7-H1 promoted local RT effect and reduced tumor burden.

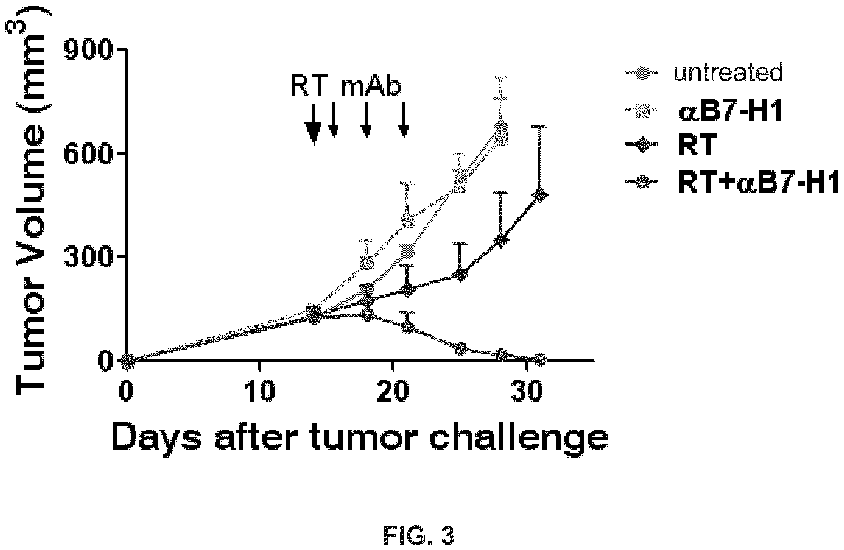

In order to test whether or not RT-mediated B7-H1 impairs acquired immune response, B7-H1/PD1 signaling pathway was blocked while performing RT. 5×10⁵ TUBO cells were injected subcutaneously in the flanks of Balb/c mice. At day 14, the mice were locally treated with a dosage of 12 Gy of irradiation (using an X-ray generator, PCM 1000, Pantak). At days 15, 18 and 21, the mice were intraperitoneally injected with 50 µg of B7-H1 blocking monoclonal antibody (clone 10F.9G2, purchased from Bio-X cell, West Lebanon, NH03784, USA), respectively, and tumor growth was monitored. The results showed that although neither RT nor the B7-H1 blocking monoclonal antibody alone had any significant effect on tumor growth, the combination of RT and the antibody generated a synergistic effect, effectively causing significant tumor regression **(****FIG. 3****)**.

### References:

1. Lee, Y., et al., Therapeutic effects of ablative radiation on local tumor require CD8+ T cells: changing strategies for cancer treatment. Blood 114, 589-595 (2009).
2. Burnette, B., et al., The efficacy of radiotherapy relies upon induction of type I interferon-dependent innate and adaptive immunity. Cancer Res.
3. Apetoh, L., et al., Toll-like receptor 4-dependent contribution of the immune system to anticancer chemotherapy and radiotherapy. Nat Med 13, 1050-1059 (2007).
4. Asano, K., et al., CD169-positive macrophages dominate antitumor immunity by crosspresenting dead cell-associated antigens. Immunity 34, 85-95.
5. Zou, W. & Chen, L., Inhibitory B7-family molecules in the tumor microenvironment. Nat Rev Immunol 8, 467-477 (2008).
6. Chen, L., Co-inhibitory molecules of the B7-CD28 family in the control of T-cell immunity. Nat Rev Immunol 4, 336-347 (2004).
7. Dong, H., et al., Tumor-associated B7-H1 promotes T-cell apoptosis: a potential mechanism of immune evasion. Nat Med 8, 793-800 (2002).
8. Curiel, T.J.,et al., Blockade of B7-H1 improves myeloid dendritic cell-mediated antitumor immunity. Nat Med 9, 562-567 (2003).
9. Barber, D.L., et al., Restoring function in exhausted CD8 T cells during chronic viral infection. Nature 439, 682-687 (2006).
10. Day, C.L., et al., PD-1 expression on HIV-specific T cells is associated with T-cell exhaustion and disease progression. Nature 443, 350-354 (2006).
11. Trautmann, L., et al., Upregulation of PD-1 expression on HIV-specific CD8+ T cells leads to reversible immune dysfunction. Nat Med 12, 1198-1202 (2006).
12. Nakamoto, N., et al., Synergistic reversal of intrahepatic HCV-specific CD8 T cell exhaustion by combined PD-1/CTLA-4 blockade. PLoSPathog 5, e1000313 (2009).
13. Freeman, G.J., Wherry, E.J., Ahmed, R. & Sharpe, A.H., Reinvigorating exhausted HIV-specific T cells via PD-1-PD-1 ligand blockade. J Exp Med 203, 2223-2227 (2006).
14. Sakuishi, K., et al., Targeting Tim-3 and PD-1 pathways to reverse T cell exhaustion and restore anti-tumor immunity. J Exp Med 207, 2187-2194.
15. Fourcade, J., et al., Upregulation of Tim-3 and PD-1 expression is associated with tumor antigen-specific CD8+ T cell dysfunction in melanoma patients. J Exp Med 207,2175-2186.
16. Mumprecht, S., Schurch, C., Schwaller, J., Solenthaler, M. &Ochsenbein, A.F., Programmed death 1 signaling on chronic myeloid leukemia-specific T cells results in T-cell exhaustion and disease progression. Blood 114, 1528-1536 (2009).
17. Hirano, F., et al., Blockade of B7-H1 and PD-1 by monoclonal antibodies potentiates cancer therapeutic immunity. Cancer Res 65, 1089-1096 (2005).
18. Rovero, S., Amici, A., Carlo, E.D., Bei, R., Nanni, P., Quaglino, E., Porcedda, P., Boggio, K., Smorlesi, A., Lollini, P.L., et al. (2000). DNA vaccination against rat her-2/Neu p185 more effectively inhibits carcinogenesis than transplantable carcinomas in transgenic BALB/c mice. J. Immunol. 165, 5133-5142.
19. Watson PA, Ellwood-Yen K, King JC, Wongvipat J, Lebeau MM, Sawyers CL., Context-dependent hormone-refractory progression revealed through characterization of a novel murine prostate cancer cell line. Cancer Res. 2005 Dec 15; 65 (24): 11565-71.
20. Wasserman J., et al., Immunosuppression in irradiated breast cancer patients: in vitro effect of cyclooxygenase inhibitors. Bull N Y Acad Med. 1989; 65: 36-44.
21. Ohuchida K., et al., Radiation to mesenchymel fibroblasts increases invasiveness of pancreatic cancer cells through tumor-mesenchymel interactions. Cancer Res. 2004; 64:3215-3222.
22. Merrick A., et al., Immunosuppressive effects of radiation on human dendritic cells: reduced IL-12 production on activation and impairment of native T-cell priming. Br J Cancer. 2005; 92: 1450-1458.
23. Chen, et al., TGF-beta released by apoptotic T cells contributes to an immunosuppressive milieu. Immunity 2001; 14: 715-725.
24. Reits EA, et al., Radiation modulates the peptide repertoire, enhances MHC class I expression, and induces successful antitumor immunotherapy. J. Exp Med. 2006; 203: 1259-1271.

## Claims

1. A B7-H1 blocking antibody for use in a method of treating cancer in combination with a radiation treatment, wherein the B7-H1 blocking antibody is administered following the radiation treatment, and wherein said B7-H1 blocking antibody is an anti-B7-H1 antibody.

2. The B7-H1 blocking antibody for the use according to claim 1, which is a monoclonal antibody to B7-H1.

3. The B7-H1 blocking antibody for the use according to any one of claims 1-2, wherein the radiation treatment is X-ray irradiation.

4. The B7-H1 blocking antibody for the use according to claim 3, wherein the radiation treatment is a single or multiple X-ray irradiation.

5. The B7-H1 blocking antibody for the use according to any one of claims 3-4, wherein the radiation treatment is 1, 2, 3, 4, 5, 6 or more X-ray irradiations.

6. The B7-H1 blocking antibody for the use according to any one of claims 3-5, wherein the X-ray dosage used in each irradiation is 5-20 Gy.

7. The B7-H1 blocking antibody for the use according to claim 6, wherein the X-ray dosage used in each irradiation is 5-8, 5-12 or 5-15 Gy.

8. The B7-H1 blocking antibody for the use according to any one of claims 1-7, wherein the cancer is selected from the group consisting of breast cancer, ovarian cancer, bladder cancer, lung cancer, prostate cancer, pancreatic cancer, colon cancer and melanoma.

9. The B7-H1 blocking antibody for the use according to claim 8, wherein the cancer is breast cancer or prostate cancer.

10. The B7-H1 blocking antibody for the use according to any one of claims 1-9, wherein said method further comprises administering a chemotherapeutic agent.

11. The B7-H1 blocking antibody for the use according to any one of claims 1-10, wherein said B7-H1 blocking antibody is administered within 9 days following the radiation treatment.

## Patentansprüche

1. B7-H1 blockierender Antikörper zur Verwendung in einem Verfahren zur Behandlung von Krebs in Kombination mit einer Strahlenbehandlung, wobei der B7-H1 blockierende Antikörper nach der Strahlenbehandlung verabreicht wird und wobei der B7-H1 blockierende Antikörper ein anti-B7-H1 Antikörper ist.

2. B7-H1 blockierender Antikörper zur Verwendung nach Anspruch 1, der ein monoklonaler Antikörper gegen B7-H1 ist.

3. B7-H1 blockierender Antikörper zur Verwendung nach einem der Ansprüche 1 - 2, wobei die Strahlenbehandlung Röntgenbestrahlung ist.

4. B7-H1 blockierender Antikörper zur Verwendung nach Anspruch 3, wobei die Strahlenbehandlung eine Einzel- oder Mehrfach-Röntgenbestrahlung ist.

5. B7-H1 blockierender Antikörper zur Verwendung nach einem der Ansprüche 3 - 4, wobei die Strahlenbehandlung 1, 2, 3, 4, 5, 6 oder mehr Röntgenbestrahlungen ist.

6. B7-H1 blockierender Antikörper zur Verwendung nach einem der Ansprüche 3 - 5, wobei die in jeder Bestrahlung verwendete Röntgendosis 5 - 20 Gy ist.

7. B7-H1 blockierender Antikörper zur Verwendung nach Anspruch 6, wobei die in jeder Bestrahlung verwendete Röntgendosis 5 - 8, 5 - 12 oder 5 - 15 Gy ist.

8. B7-H1 blockierender Antikörper zur Verwendung nach einem der Ansprüche 1 - 7, wobei der Krebs ausgewählt ist aus der Gruppe bestehend aus Brustkrebs, Eierstockkrebs, Blasenkrebs, Lungenkrebs, Prostatakrebs, Bauchspeicheldrüsenkrebs, Kolonkarzinom und Melanom.

9. B7-H1 blockierender Antikörper zur Verwendung nach Anspruch 8, wobei der Krebs Brustkrebs oder Prostatakrebs ist.

10. B7-H1 blockierender Antikörper zur Verwendung nach einem der Ansprüche 1 - 9, wobei das Verfahren weiterhin Verabreichen eines chemotherapeutischen Mittels umfasst.

11. B7-H1 blockierender Antikörper zur Verwendung nach einem der Ansprüche 1 - 10, wobei der B7-H1 blockierende Antikörper innerhalb von 9 Tagen nach der Strahlenbehandlung verabreicht wird.

## Revendications

1. Anticorps bloquant B7-H1 destiné à être utilisé dans un procédé de traitement du cancer en combinaison avec un traitement par rayonnement, où l'anticorps bloquant B7-H1 est administré à la suite du traitement par rayonnement, et où ledit anticorps bloquant B7-H1 est un anticorps anti-B7-H1.

2. Anticorps bloquant B7-H1 destiné à être utilisé selon la revendication 1, qui est un anticorps monoclonal contre B7-H1.

3. Anticorps bloquant B7-H1 destiné à être utilisé selon l'une quelconque des revendications 1-2, où le traitement par rayonnement est une irradiation avec des rayons X.

4. Anticorps bloquant B7-H1 destiné à être utilisé selon la revendication 3, où le traitement par rayonnement est une irradiation avec des rayons X unique ou multiple.

5. Anticorps bloquant B7-H1 destiné à être utilisé selon l'une quelconque des revendications 3-4, où le traitement par rayonnement est 1, 2, 3, 4, 5, 6 irradiations avec des rayons X ou plus.

6. Anticorps bloquant B7-H1 destiné à être utilisé selon l'une quelconque des revendications 3-5, où la dose de rayons X utilisée dans chaque irradiation est 5-20 Gy.

7. Anticorps bloquant B7-H1 destiné à être utilisé selon la revendication 6, où la dose de rayons X utilisée dans chaque irradiation est 5-8, 5-12 ou 5-15 Gy.

8. Anticorps bloquant B7-H1 destiné à être utilisé selon l'une quelconque des revendications 1-7, où le cancer est choisi dans le groupe consistant en le cancer du sein, le cancer ovarien, le cancer de la vessie, le cancer du poumon, le cancer de la prostate, le cancer du pancréas, le cancer du côlon et le mélanome.

9. Anticorps bloquant B7-H1 destiné à être utilisé selon la revendication 8, où le cancer est le cancer du sein ou le cancer de la prostate.

10. Anticorps bloquant B7-H1 destiné à être utilisé selon l'une quelconque des revendications 1-9, où ledit procédé comprend en outre l'administration d'un agent chimiothérapeutique.

11. Anticorps bloquant B7-H1 destiné à être utilisé selon l'une quelconque des revendications 1-10, où ledit anticorps bloquant B7-H1 est administré dans les 9 jours qui suivent le traitement par rayonnement.
